# EUROPEAN PATENT SPECIFICATION

(11) **EP 3 964 126 B1**
(45) Date of publication and mention of the grant of the patent: **04.03.2026**
(21) Application number: 20827371.4
(22) Date of filing: 17.06.2020
(51) Int. Cl.: A61B 5/024, A61B 5/00

(54) **PHOTOPLETHYSMOGRAPH AND TERMINAL**
FOTOPLETHYSMOGRAF UND ENDGERÄT
PHOTOPLÉTHYSMOGRAPHE ET TERMINAL

(30) Priority: 17.06.2019 CN 201910521035
(43) Date of publication of application: 09.03.2022
(73) Proprietor: Huawei Technologies Co., Ltd., Shenzhen, Guangdong 518129 (CN)
(72) Inventor: YANG, Rongguang, Shenzhen, Guangdong 518129 (CN); XI, Yi, Shenzhen, Guangdong 518129 (CN); SUN, Shiyou, Shenzhen, Guangdong 518129 (CN); GUO, Zhi, Shenzhen, Guangdong 518129 (CN); ZHANG, Bin, Shenzhen, Guangdong 518129 (CN)
(74) Representative: Gill Jennings & Every LLP
(86) International application number: PCT/CN2020/096460
(87) International publication number: WO 2020/253697

(56) References cited:
- CN-A- 106 793 966
- CN-A- 107 106 036
- CN-A- 107 405 092
- CN-A- 109 171 653
- CN-A- 109 363 628
- CN-A- 109 363 628
- CN-A- 110 432 883
- CN-U- 205 625 904
- US-A1- 2016 029 911
- US-A1- 2016 073 954
- US-A1- 2017 086 691
- US-A1- 2017 224 236
- US-A1- 2018 014 781
- US-A1- 2018 325 397
- US-A1- 2019 171 165

## Description

### TECHNICAL FIELD

Embodiments of this application relate to the field of intelligent terminal technologies, and in particular, to a photoplethysmography sensor and a terminal.

### BACKGROUND

A photoplethysmography sensor can detect a sport heart rate of a human body by using a photoplethysmography (photoplethysmograph, PPG for short) technology, can detect a difference of intensity of light reflected after absorption performed by blood and tissue of a human body, and record changes of a vessel volume during a cardiac cycle, so that a heart rate can be calculated from a pulse waveform. The photoplethysmography sensor is an application of an infrared nondestructive detection technology in biomedicine.

The photoplethysmography sensor is disposed in an existing smartwatch or smart band, and heart rate detection may be performed by using the photoplethysmography sensor. The photoplethysmography sensor includes an optical device and a photoelectric sensor. Light may be emitted to a human body by using the optical device, and light reflected after absorption performed by blood and tissue of the human body is detected by using the photoelectric sensor to detect a change of a heart rate of a wearer.

However, the light emitted by the optical device is very likely to leak, and consequently, a light crossover between the optical device and the photoelectric sensor is severe. This affects heart rate measurement precision and affects user experience.

US2017086691A1 describes light-blocking structures for optical physiological parameter measurement devices or sensors.

US2017224236A1 describes a wearable apparatus and a photoplethysmograph sensor unit.

US2016073954A1 describes a wristwatch including an integrated pulse oximeter.

### SUMMARY

Embodiments of this application provide a photoplethysmography sensor and a terminal, so as to ensure heart rate measurement precision and also resolve a problem of a light crossover inside the photoplethysmography sensor.

To achieve the foregoing objectives, the following technical solutions are used in the embodiments of this application:
According to a first aspect of the present invention, a photoplethysmography sensor is provided, including: a housing, a cover plate, an optical device, configured to emit light outwards, and a photoelectric sensor, configured to receive an external optical signal. The housing and the cover plate form enclosed space, and the optical device and the photoelectric sensor are accommodated in the enclosed space. The cover plate includes a first area used by the optical device to emit the light outwards and a second area used by the photoelectric sensor to receive the external optical signal. The cover plate further includes a third area, a shielding structure is disposed on the third area, and the shielding structure is configured to isolate light between the optical device and the photoelectric sensor. Therefore, the shielding structure is disposed in the third area, thereby improving isolation between the optical device and the photoelectric sensor, avoiding a light crossover between the optical device and the photoelectric sensor, improving measurement precision of the photoelectric sensor, and improving user experience.

The photoplethysmography sensor further includes a light insulating plate, the light insulating plate is disposed around the optical device, and one end of the light insulating plate is connected to the cover plate. The light insulating plate is configured to isolate the optical device and the photoelectric sensor, thereby further avoiding the light crossover between the optical device and the photoelectric sensor in the enclosed space.

In an optional implementation, the shielding structure includes a groove, and the groove is disposed around the first area. Therefore, a thickness of the cover plate at a position of the groove is less than a thickness of the cover plate at another position, and a light propagation path changes at the position of the groove, thereby reducing total reflection of light at the groove, and improving the light crossover between the optical device and the photoelectric sensor.

In an optional implementation, a depth of the groove is 0.002 mm to 0.2 mm. Therefore, a proper groove depth may be selected provided that strength of the cover plate is not affected, and this is conducive to improving the light crossover between the optical device and the photoelectric sensor, and improving the isolation between the optical device and the photoelectric sensor.

In an optional implementation, an inner surface of the groove is provided with a grain. Therefore, roughness of the inner surface of the groove is increased, a specular reflection phenomenon at the position of the groove is reduced, the light crossover between the optical device and the photoelectric sensor is further improved, and the isolation between the optical device and the photoelectric sensor is improved.

In an optional implementation, the inner surface of the groove is covered with a coating for absorbing light. Therefore, when light emitted by the optical device reaches the position of the groove, the light is absorbed by the coating, thereby avoiding the light crossover between the optical device and the photoelectric sensor.

In an optional implementation, the third area is also covered with a coating for absorbing light, and the coating is located on a side, of the third area, facing the housing. Therefore, when light emitted by the optical device reaches the third area, the light is absorbed by the coating, thereby preventing the light of the optical device from leaking out of the housing through the cover plate, and avoiding user experience deterioration caused by blinding due to light leakage.

In an optional implementation, the coating is black ink. Therefore, light that reaches the coating can be fully absorbed, a light shielding effect can be enhanced, and the cover plate does not need to be carved, thereby ensuring completeness of appearance of the cover plate.

According to the invention, the shielding structure includes a light-shielding ring, the light-shielding ring is disposed around the first area, the light-shielding ring is carved through a laser process, and the light-shielding ring is injected with a light-shielding material. Therefore, while impact-resistance performance of the cover plate is ensured, the light crossover between the optical device and the photoelectric sensor is improved.

In an optional implementation, the housing includes a bottom housing and a convex part, the bottom housing and the convex part are integrally formed, the cover plate is flat-shaped, the convex part is provided with a first opening, and the cover plate is clamped to the first opening. Therefore, the housing uses an integrally-forming process, has higher strength, and can withstand external impact, and the cover plate may be formed into a flat shape, so that a height of the cover plate is less than a height of the surrounding housing, and the surrounding housing can bear as much external impact as possible, thereby improving the impact-resistance performance of the cover plate.

In an optional implementation, the housing includes a bottom housing, the bottom housing is provided with a second opening, the cover plate is convex, and the cover plate is clamped to the second opening. Therefore, the housing has a simple structure, and this reduces processing difficulty, and is conducive to mass production.

In an optional implementation, a recess is provided on a side, of the cover plate, facing the optical device, and the optical device is disposed in the recess. Therefore, a thickness of the cover plate at the recess is relatively small, a height difference is generated between the area of the cover plate at the recess and a cover plate area corresponding to the photoelectric sensor, and light emitted by the optical device may be directly emitted through the cover plate at the recess, thereby further avoiding the light crossover between the optical device and the photoelectric sensor, and improving the isolation between the optical device and the photoelectric sensor.

In an optional implementation, a side, of the cover plate, facing away from the optical device is provided with a planar face, and the planar face is opposite to the recess. Therefore, the cover plate at the recess is set to a flat shape, so that a height of the position may be less than a height of a surrounding cover plate, and a surrounding thick cover plate may bear as much external impact as possible, thereby improving the impact-resistance performance of the cover plate.

In an optional implementation, the optical device is fastened in the recess through glue injection. Therefore, the optical device and the cover plate are injection-molded into an integrated module, thereby improving strength of the cover plate at the position of the recess, enhancing the impact-resistance performance of the cover plate, improving stability of the optical device, and avoiding fall-off of the optical device caused by external impact.

In an optional implementation, the second area includes one or more light transmission windows, each light transmission window corresponds to one photoelectric sensor, and the light transmission windows are uniformly arranged around the first area. Therefore, the photoelectric sensor can fully receive light emitted from the optical device and reflected from a to-be-detected part of a human body, thereby improving working efficiency of the photoplethysmography sensor.

In an optional implementation, a material of the housing is ceramic or plastic. Therefore, the housing may be integrally formed by injection molding, thereby enhancing strength of the housing, and improving impact-resistance performance of the housing.

In an optional implementation, a material of the cover plate is sapphire or toughened glass. Therefore, the strength of the cover plate is enhanced and the impact-resistance performance of the cover plate is improved.

According to a second aspect of the embodiments of this application, a terminal is provided. The terminal includes a body and a fastening part, the body includes the photoplethysmography sensor described above, the fastening part is configured to fasten the body to a to-be-detected body part of a user, and when the body is fastened to the to-be-detected body part of the user, the cover plate of the photoplethysmography sensor faces the to-be-detected body part. Therefore, the terminal uses the photoplethysmography sensor. This avoids blinding due to light leakage, avoids an internal light crossover phenomenon, and improves user experience.

In an optional implementation, the terminal is a smartwatch, a smart band, or a smartphone. Therefore, the photoplethysmography sensor is integrated into the terminal, and a heart rate change status of a wearer can be detected provided that the wearer wears the terminal, thereby improving user experience.

### BRIEF DESCRIPTION OF DRAWINGS

FIG. 1 is a schematic structural diagram of a terminal according to an embodiment of this application;
FIG. 2 is an exploded view of a photoplethysmography sensor according to an embodiment of this application;
FIG. 3 is a cross-sectional view of a photoplethysmography sensor according to an embodiment of this application;
FIG. 4 is a schematic structural diagram of a cover plate according to an embodiment of this application;
FIG. 5 is a schematic structural diagram of another cover plate according to an embodiment of this application;
FIG. 6 is a schematic structural diagram of another cover plate according to an embodiment of this application;
FIG. 7 is a schematic structural diagram of another cover plate according to an embodiment of this application;
FIG. 8 is a schematic structural diagram of another photoplethysmography sensor according to an embodiment of this application;
FIG. 9 is a schematic structural diagram of another photoplethysmography sensor according to an embodiment of this application; and
FIG. 10 is a structural block diagram of a terminal according to an embodiment of this application.

### DESCRIPTION OF EMBODIMENTS

The following describes the technical solutions in the embodiments of this application with reference to the accompanying drawings in the embodiments of this application. To clearly describe the technical solutions in the embodiments of this application, terms such as "first" and "second" are used in the embodiments of this application to distinguish between same items or similar items that have basically the same functions or purposes. A person skilled in the art may understand that the terms such as "first" and "second" do not limit a quantity or an execution sequence, and the terms such as "first" and "second" do not mean being definitely different either.

To facilitate understanding of a photoplethysmography sensor provided in the embodiments of this application, the following first describes an application scenario of the photoplethysmography sensor. The photoplethysmography sensor may be applied to a terminal, for example, a common electronic device such as a mobile phone, a tablet computer, a digital camera, or a wearable device. An optical device in the photoplethysmography sensor may emit light outwards, a part of the light is reflected after absorption performed by blood and tissue of a human body of a user, and a photoelectric sensor in the photoplethysmography sensor may receive light reflected from the human body of the user. Further, a change status such as a heart rate of a wearer can be determined based on reflected light received by the photoelectric sensor.

Usually, in the terminal, the optical device and the photoelectric sensor are disposed adjacent to each other. When the optical device is used, light emitted by the optical device is likely to leak to cause blinding, and this affects user experience. In addition, a light crossover between the optical device and the photoelectric sensor is severe, and this affects power consumption of the entire system of the terminal and heart rate measurement precision.

To this end, an embodiment of this application provides an improved terminal.

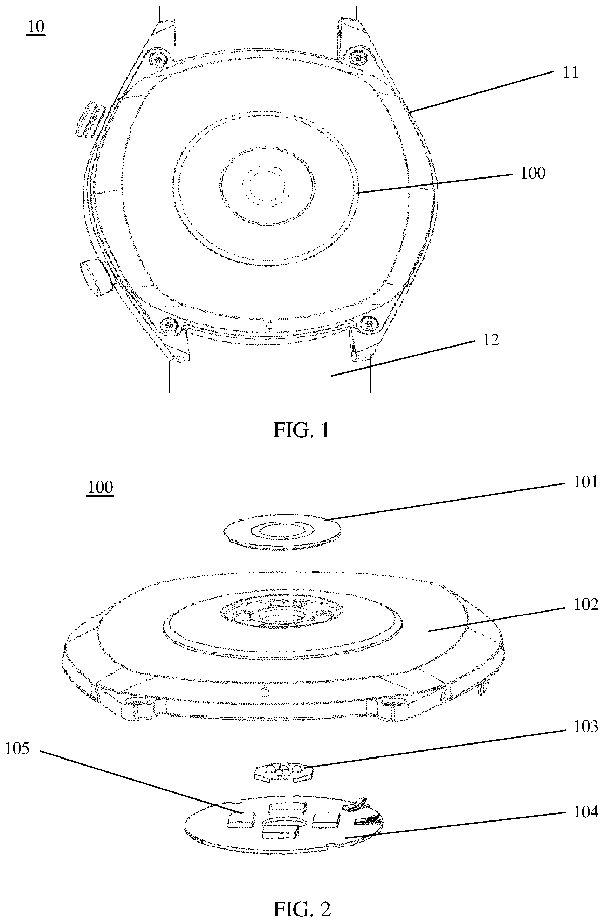

The following describes the terminal provided in this embodiment of this application with reference to the accompanying drawings. FIG. 1 is a schematic structural diagram of a terminal according to an embodiment of this application. As shown in FIG. 1, the terminal 10 includes a body 11 and a fastening part 12. The body 11 is fixedly connected to the fastening part 12. The body 11 includes a photoplethysmography sensor, the fastening part is configured to fasten the body 11 to a to-be-detected body part of a user, and when the body 11 is fastened to the to-be-detected body part of the user, the photoplethysmography sensor faces the to-be-detected body part.

For example, the terminal is, for example, a smartwatch, the body is a watch face, the fastening part is a watch band, the photoplethysmography sensor is, for example, disposed on a back surface of the watch face, and when the user uses the smartwatch, a human body of the user is in contact with the back surface of the watch face. The photoplethysmography sensor faces the to-be-detected body part of the user.

Therefore, the photoplethysmography sensor is integrated into the smartwatch, and a heart rate change status of a wearer can be detected provided that the wearer wears the terminal, thereby improving user experience.

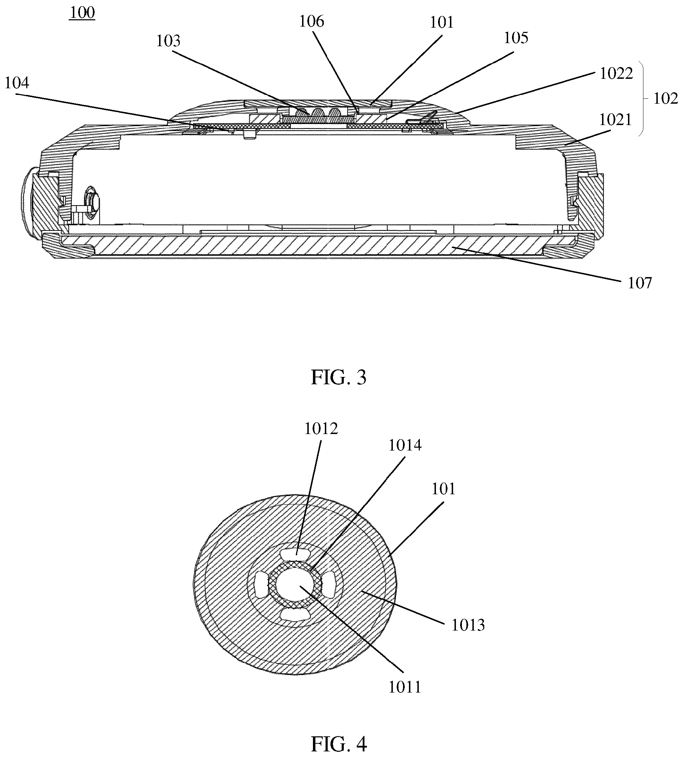

FIG. 2 is an exploded view of a photoplethysmography sensor according to an embodiment of this application. FIG. 3 is a cross-sectional view of a photoplethysmography sensor according to an embodiment of this application. As shown in FIG. 2 and FIG. 3, the photoplethysmography sensor 100 includes a housing 102, a cover plate 101, an optical device 103, configured to emit light outwards, and a photoelectric sensor 105, configured to receive an external optical signal. The housing 102 and the cover plate 101 form enclosed space, and the optical device 103 and the photoelectric sensor 105 are accommodated in the enclosed space.

Specific structures of the optical device 103 and the photoelectric sensor 105 are not limited in this embodiment of this application. For example, the optical device 103 may be, for example, a light emitting diode, and the photoelectric sensor 105 may be, for example, a photodiode. When the photoplethysmography sensor works, the optical device may emit light outwards, a part of the light is reflected after absorption performed by blood and tissue of a human body of a user, and the photoelectric sensor may receive light reflected from the human body of the user.

A specific material of the cover plate 101 is not limited in this embodiment of this application, provided that light transmission requirements of the optical device 103 and the photoelectric sensor 105 can be met. In a specific implementation of this application, a material of the cover plate 101 is sapphire or toughened glass. The sapphire and the toughened glass have good light transmission and relatively high strength, and improve impact-resistance performance of the cover plate while ensuring the light transmission.

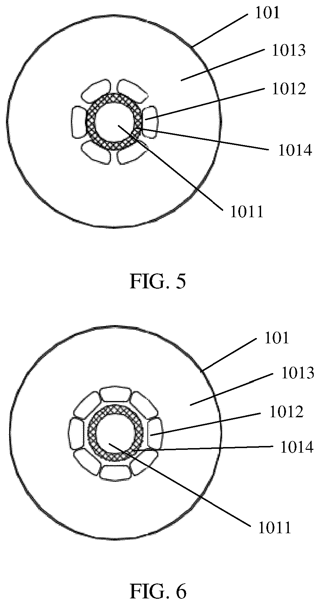

A specific structure of the cover plate 101 is not limited in this embodiment of this application. The cover plate 101 includes a first surface and a second surface that are opposite to each other. For example, FIG. 4 shows the first surface of the cover plate 101, and FIG. 5, FIG. 6, and FIG. 7 show the second surface of the cover plate. The first surface faces the housing 102 and forms the enclosed space with the housing 102, and the second surface faces away from the housing 102.

As shown in FIG. 4, FIG. 5, FIG. 6, and FIG. 7, the cover plate 101 may be divided into a first area 1011 and a second area 1012 based on fields of vision (fields of vision, FOVs for short) of the optical device 103 and the photoelectric sensor 105 on the cover plate 101. For example, a field of vision of the optical device 103 on the cover plate 101 is the first area 1011, and the optical device 103 transmits an optical signal through the first area 1011. A field of vision of the photoelectric sensor 105 on the cover plate 101 is the second area 1012, and the photoelectric sensor 105 receives an optical signal through the second area 1012.

There are relatively high requirements on light transmission of the first area 1011 and the second area 1012. A structure that can enhance light transmission of the two may be disposed in the first area 1011 and the second area 1012, or no processing needs to be performed.

A quantity, a shape, and a position of the first area 1011 and the second area 1012 are not limited in this embodiment of this application. The first area 1011 includes, for example, one first light transmission window, the second area 1012 includes, for example, one or more second light transmission windows, and the second light transmission windows are disposed around the first light transmission window. The first light transmission window is, for example, circular or square, and the second light transmission window is in a concentric ring shape, a square shape, or an irregular shape.

For example, as shown in FIG. 4, the first area 1011 includes, for example, one first light transmission window, the first light transmission window is circular, and the first light transmission window is located in the center of the cover plate 101. The second area 1012 includes four second light transmission windows, the second light transmission windows are in an irregular shape, each second light transmission window corresponds to one photoelectric sensor 105, and the second light transmission windows are evenly arranged around the first light transmission window.

Therefore, the photoelectric sensor can fully receive light emitted from the optical device and reflected from a to-be-detected part of a human body, thereby improving working efficiency of the photoplethysmography sensor.

As shown in FIG. 5, the first area 1011 includes one first light transmission window, the first light transmission window is circular, and the first light transmission window is located in the center of the cover plate 101. The second area 1012 includes six second light transmission windows, the second light transmission windows are in an irregular shape, and the second light transmission windows are evenly arranged around the first light transmission window.

As shown in FIG. 6, the first area 1011 includes one first light transmission window, the first light transmission window is circular, and the first light transmission window is located in the center of the cover plate 101. The second area 1012 includes eight second light transmission windows, the second light transmission windows are in an irregular shape, and the second light transmission windows are evenly arranged around the first light transmission window.

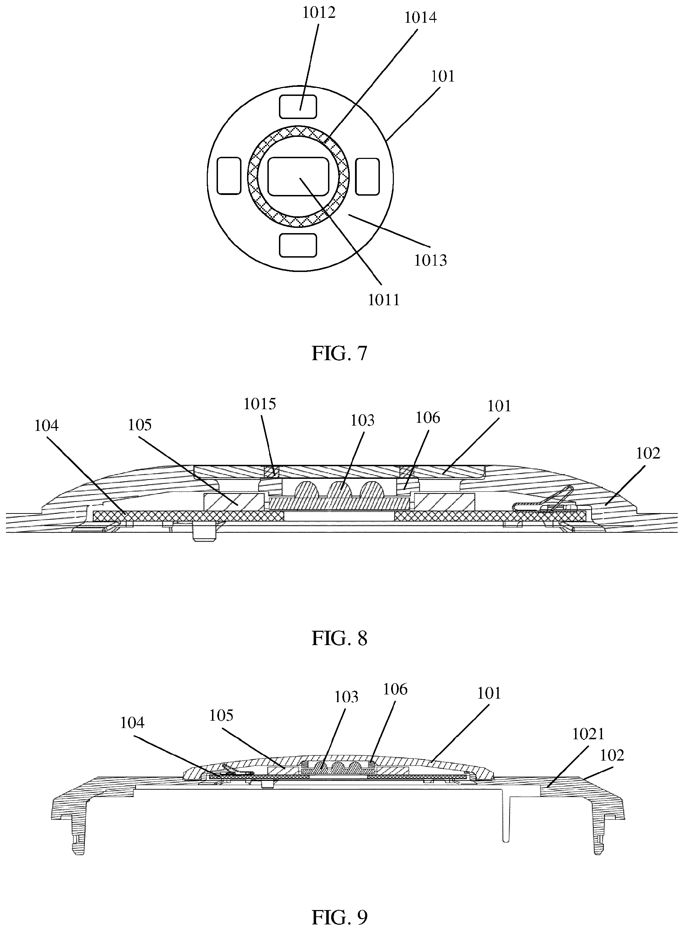

As shown in FIG. 7, the first area 1011 includes one first light transmission window, the first light transmission window is square, and the first light transmission window is located in the center of the cover plate 101. The second area 1012 includes four second light transmission windows, the second light transmission windows are square, and the second light transmission windows are evenly arranged around the first light transmission window.

The cover plate 101 further includes, for example, a third area 1013, and the third area 1013 is an area on the cover plate 101 other than the first area 1011 and the second area 1012. For example, the cover plate 101 is made of glass. When light emitted by the optical device 103 reaches the cover plate 101, due to different refractive indexes of the glass and air, a total reflection phenomenon occurs in the cover plate 101. In addition, because a surface of the cover plate 101 is relatively smooth, specular reflection occurs.

The total reflection phenomenon and the specular reflection phenomenon cause a part of the light emitted by the optical device 103 to directly enter a collection range of the photoelectric sensor 105 without passing through the to-be-detected human body part, thereby affecting detection accuracy of the photoelectric sensor 105.

In order to improve measurement precision of the photoelectric sensor 105, for example, a shielding structure is disposed on the third area 1013, and the shielding structure is configured to isolate light between the optical device 103 and the photoelectric sensor 105, to prevent light that reaches the first area 1011 of the cover plate 101 from entering the second area 1012 through the third area 1013, and prevent light that reaches the second area 1012 from reaching the first area 1011 through the third area 1013, thereby improving isolation between the optical device 103 and the photoelectric sensor 105, and improving measurement precision of the photoelectric sensor 105.

According to the photoplethysmography sensor provided in this embodiment of this application, the shielding structure is disposed in the third area, so that the isolation between the optical device and the photoelectric sensor is improved, the light crossover between the optical device and the photoelectric sensor is avoided, the measurement precision of the photoelectric sensor is improved, and user experience is improved.

A specific structure of the shielding structure is not limited in this embodiment of this application. In a specific implementation of this application, as shown in FIG. 4, FIG. 5, FIG. 6, and FIG. 7, the shielding structure includes, for example, a groove 1014. The groove 1014 is located between the first area 1011 and the second area 1012, and the groove 1014 is disposed around the first area 1011. Therefore, when light emitted by the optical device 103 reaches a position of the groove 1014, a thickness of the cover plate 101 at the position of the groove 1014 is less than a thickness of the cover plate at another position, and a light propagation path changes at the position, thereby reducing total reflection of light at the groove 1014, and improving the light crossover between the optical device 103 and the photoelectric sensor 105.

A specific structure of the groove 1014 is not limited in this embodiment of this application. In a specific implementation of this application, the groove 1014 is located in the third area 1013 of the first surface of the cover plate 101. In another implementation of this application, the groove 1014 is located in the third area 1013 of the second surface of the cover plate 101. In other implementations of this application, the groove 1014 is disposed in both the third area 1013 of the first surface and the third area 1013 of the second surface of the cover plate 101, and the groove of the first surface and the groove of the second surface are opposite to each other.

A depth of the groove 1014 is, for example, 0.002 mm to 0.2 mm, and the groove 1014 may be formed on the cover plate 101 by, for example, laser carving or groove milling. Therefore, a proper groove depth may be selected provided that strength of the cover plate is not affected, and this is conducive to improving the light crossover between the optical device and the photoelectric sensor, and improving the isolation between the optical device and the photoelectric sensor.

For example, an inner surface of the groove 1014 is provided with a grain. For example, a rough grained-surface ring is disposed on the inner surface of the groove 1014, and this increases roughness of the inner surface of the groove 1014, reduces a specular reflection phenomenon at the position of the groove 1014, and further improves the light crossover between the optical device 103 and the photoelectric sensor 105.

The inner surface of the groove 1014 is also covered with, for example, a coating for absorbing light, the coating may be in a relatively dark color such as black, and the coating may cover the inner surface of the groove 1014 by printing, evaporation, or spraying. For example, the coating is, for example, black ink. For example, the black ink may cover the inner surface of the groove 1014 by spraying.

A disposing range of the groove 1014 is not limited in this embodiment of this application. For example, a range of the groove 1014 is less than or equal to a range of the third area 1013. If the range of the groove 1014 is equal to the range of the third area 1013, the groove 1014 is disposed in all the third areas 1013. If the range of the groove 1014 is less than the range of the third area 1013, all areas of the third area 1013 other than the groove 1014 are covered with, for example, a coating for absorbing light, and the coating may be formed by using the same material and process as those of the coating in the groove 1014. The light crossover between the optical device 103 and the photoelectric sensor 105 is further improved, and the isolation between the optical device 103 and the photoelectric sensor 105 is improved.

As shown in FIG. 4, in an implementation of this application, the coating covers the third area 1013 of the first surface of the cover plate 101. Therefore, when light emitted by the optical device 103 reaches the third area 1013, the light is absorbed by the coating, thereby avoiding the light crossover between the optical device 103 and the photoelectric sensor 105, also preventing the light of the optical device 103 from leaking out of the housing 102 through the cover plate 101, and avoiding user experience deterioration caused by blinding due to light leakage.

In another implementation of this application, the third area 1013 of the second surface of the cover plate 101 is also covered with the coating. Therefore, incidence of other light in an external environment is avoided, impact of the external environment on the photoplethysmography sensor is reduced, and the measurement precision of the photoelectric sensor 105 is improved.

However, the cover plate at the position of the groove is relatively thin and impact-resistance performance is poor. To this end, an embodiment of this application provides a new shielding structure. As shown in FIG. 8, the shielding structure includes, for example, a light-shielding ring 1015, and the light-shielding ring is injected with a light-shielding material. For a specific position of the light-shielding ring 1015, refer to the groove. Details are not described herein. Processing of the light-shielding ring includes, for example, the following steps: first, deep micro carving is performed on the cover plate 101 through a laser process, and then the black light-shielding material is injected to form a black ring-shaped light-shielding structure. In this way, while the impact-resistance performance of the cover plate is ensured, the light crossover between the optical device 103 and the photoelectric sensor 105 is improved.

In this embodiment of this application, a disposing range of the light-shielding ring 1015 is not limited. For example, a range of the light-shielding ring 1015 is less than or equal to a range of the third area. If the range of the light-shielding ring 1015 is equal to the range of the third area 1013, the foregoing light-shielding processing is performed on all the third areas. If the range of the light-shielding ring 1015 is less than the range of the third area 1013, no processing may be performed on an area in the third area other than the light-shielding ring 1015, or the area may be covered with a layer of coating for absorbing light as described above.

To further improve isolation of the photoplethysmography sensor, as shown in FIG. 3, FIG. 8, and FIG. 9, for example, a light insulating plate 106 is further disposed in the housing 102, and the light insulating plate 106 is disposed around the optical device 103. One end of the light insulating plate 106 extends to a root of the optical device 103, and the other end is connected to the cover plate 101.

In an implementation of this application, the light insulating plate 106 and the housing 102 are integrally formed.

In another implementation of this application, the light insulating plate 106 is an independent part, and the light insulating plate 106 may be assembled around the optical device 103. The light insulating plate is configured to isolate the optical device 103 and the photoelectric sensor 105, so as to avoid a light crossover between the optical device 103 and the photoelectric sensor 105 in the enclosed space.

In this embodiment of this application, a specific material of the light insulating plate is not limited. The light insulating plate may be made of any non-transparent material, provided that the light insulating plate may implement light blocking.

A structure of the housing 102 is not limited in this embodiment of this application. In an implementation of this application, as shown in FIG. 3, the housing 102 includes a bottom housing 1021 and a convex part 1022. The bottom housing 1021 and the convex part 1022 are integrally formed. The cover plate 101 is flat-shaped, a first opening is disposed on the convex part 1022, and the cover plate 101 is clamped to the first opening.

For example, a support component is further disposed on the convex part 1022 at a position of the first opening. For example, the cover plate 101 is fixedly connected to the support component in an adhesive dispensing manner.

For example, the bottom housing 1021 and the convex part 1022 may be integrally formed by injection molding without cutting or the like, thereby reducing processing costs of the part. Certainly, the bottom housing 1021 and the convex part 1022 may be separately formed, and then assembled into a whole by using a lamination process.

In another implementation of this application, as shown in FIG. 9, the housing 102 includes a bottom housing 1021, a second opening is disposed on the bottom housing 1021, the cover plate 101 is convex, and the cover plate 101 is clamped to the second opening.

For example, a support component is further disposed on the bottom housing 1021 at a position of the second opening. For example, the cover plate 101 is fixedly connected to the support component in an adhesive dispensing manner.

For example, the cover plate 101 may be integrally formed by injection molding, and a protrusion does not need to be disposed on the bottom housing 1021, thereby reducing process difficulty.

Next, referring to FIG. 9, for example, a recess is further disposed on a side, of the cover plate 101, facing the optical device 103, and the optical device 103 is disposed in the recess. Therefore, a thickness of the cover plate at the recess is relatively small, a height difference is generated between the area of the cover plate at the recess and a cover plate area corresponding to the photoelectric sensor, and light emitted by the optical device may be directly emitted through the cover plate at the recess, thereby further avoiding the light crossover between the optical device and the photoelectric sensor, and improving the isolation between the optical device and the photoelectric sensor.

A side, of the cover plate 101, facing away from the optical device 103 is provided with a planar face, and the planar face is opposite to the recess. Therefore, a position that is of the second surface of the cover plate 101 and that is opposite to the recess is flattened, so that a height of the position may be less than a height of a surrounding cover plate 101, and a surrounding thick cover plate 101 may bear as much external impact as possible, thereby improving the impact-resistance performance of the cover plate 101.

The optical device 103 is fastened in the recess through glue injection. For example, the optical device 103 may be glue-injected and sealed in the recess by using transparent adhesive. After solidification, the optical device 103 and the cover plate 101 form an integrated module, thereby improving strength of the cover plate 101 at the position of the recess, improving stability of the optical device 103, and avoiding fall-off of the optical device 103 caused by external impact.

A material of the housing 102 is not limited in this embodiment of this application, and a material of the housing 102 is ceramic or plastic. For example, the housing 102 may be integrally formed by injection molding.

Next, referring to FIG. 2, the photoplethysmography sensor further includes, for example, a PCB board 104, and the circuit board is located in the enclosed space and fixedly disposed on the housing 102. The optical device 103 and the photoelectric sensor 105 may be fastened onto the PCB 104, for example, in a welding or cementing manner, where the PCB 104 may be a common printed circuit board.

In another implementation of this application, the PCB includes, for example, a first PCB board and a second PCB board, the optical device is fastened onto the first PCB board, the photoelectric sensor is fastened onto the second PCB board, for example, the first PCB board is circular, and for example, the second PCB board is annular. The first PCB board is fastened onto the second PCB board, and the first PCB board is electrically connected to the second PCB board.

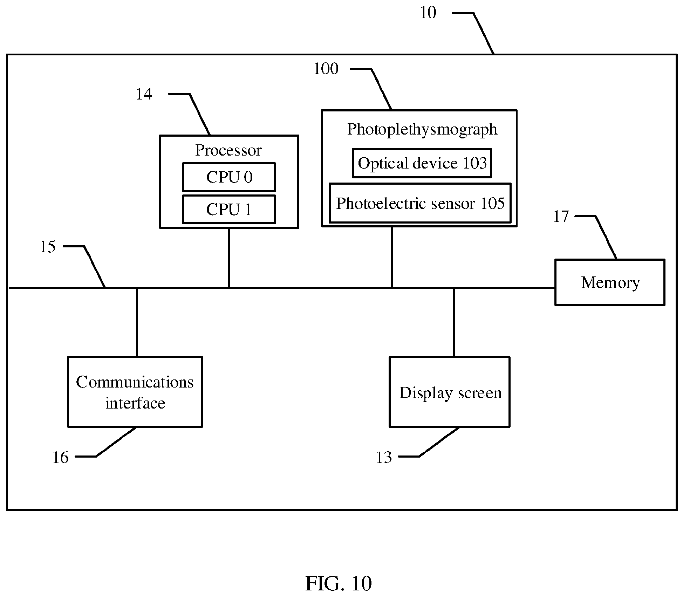

FIG. 10 is a structural block diagram of a terminal according to an embodiment of this application. The terminal further includes a display screen 13, at least one processor 14, a communications bus 15, at least one communications interface 16, and a memory 17. It may be understood that the terminal in FIG. 10 is merely an example of the terminal, and does not constitute any limitation on the terminal. The terminal may include more or fewer components than those shown in the figure, or combine some components, or have different components. Although not shown, the terminal may further include a battery, a camera, a Bluetooth module, a global positioning system (global positioning system, GPS) module, and the like. Details are not described herein.

The processor 14 is communicatively connected to the at least one communications interface 16, the memory 17, and the display screen 13 by using the communications bus 15. The processor 14 may be a central processing unit (Central Processing Unit, CPU), or may be another general purpose processor 14, a digital signal processor 14 (Digital Signal Processor, DSP), an application-specific integrated circuit (Application-Specific Integrated Circuit, ASIC), a field-programmable gate array (Field-Programmable Gate Array, FPGA) or another programmable logic device, a discrete gate or transistor logic device, or a discrete hardware component. The general-purpose processor 14 may be a microprocessor 14, or the processor 14 may be any conventional processor 14, or the like. The processor 14 is a control center of the terminal, and connects all parts of the entire terminal by using various interfaces and lines.

The display screen 13 may be configured to display information entered by a user or information provided for a user, and various menus of the terminal. The display screen 13 may be in a form of a liquid crystal display (liquid crystal display, LCD), an organic light-emitting diode (organic light-emitting diode, OLED), or the like.

The communications bus 15 may include a path to transmit information between the foregoing components.

The communications interface 16 is any apparatus such as a transceiver, and is configured to communicate with another device or a communications network, such as the Ethernet, a radio access network (radio access network, RAN), or a wireless local area network (wireless local area network, WLAN).

The memory 17 may be configured to store a computer program and/or a module. The processor 14 implements various functions of the terminal by running or executing the computer program and/or the module stored in the memory 17 and invoking data stored in the memory 17. The memory 17 may mainly include a program storage area and a data storage area. The program storage area may store an operating system, an application program (such as a sound playing function or an image playing function) that is required by a plurality of functions, and the like. The data storage area may store data (such as audio data or a phone book) that is created based on use of the terminal, and the like. In addition, the memory 17 may include a high-speed random access memory 17, and may further include a non-volatile memory 17, for example, a hard disk, a memory, a plug-in hard disk, a smart media card (smart media card, SMC), a secure digital (secure digital, SD) card, a flash card (flash card), a plurality of magnetic disk storage devices 17, a flash memory, or another volatile solid-state storage device 17. The memory 17 may exist independently, and be connected to the processor 14 by using the communications bus 15. Alternatively, the memory 17 may be integrated with the processor 14.

During specific implementation, in an embodiment, the processor 14 may include one or more CPUs, for example, a CPU 0 and a CPU 1 in the figure.

During specific implementation, in an embodiment, the terminal may include a plurality of processors 14, for example, the processor 14 in the figure and a processor 141. Each of these processors 14 may be a single-core (single-CPU) processor 14 or may be a multi-core (multi-CPU) processor 14. The processor 14 herein may be one or more devices, circuits, and/or processing cores used to process data (for example, a computer program instruction).

In the embodiment of this application, the processor 14 is separately connected to the optical device 103 and the photoelectric sensor 105, and when the user triggers the photoplethysmography sensor to start working, the processor 14 controls the optical device 103 to emit light outwards, a part of the light is reflected after absorption performed by blood and tissue of a human body of the user, and the photoelectric sensor 105 may receive light reflected from the human body of the user, convert the optical signal into an electrical signal, and send the electrical signal to the processor 14. The processor 14 can determine a change status such as a heart rate of a wearer based on the reflected light received by the photoelectric sensor 105, and display the change status by using the display screen 13.

The foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

## Claims

1. A photoplethysmography sensor, comprising: a housing (102), a cover plate (101), an optical device (103), configured to emit light outwards, and a photoelectric sensor (105), configured to receive an external optical signal, wherein
the housing and the cover plate form enclosed space, and the optical device and the photoelectric sensor are accommodated in the enclosed space;
the cover plate comprises a first area (1011) used by the optical device to emit the light outwards and a second area (1012) used by the photoelectric sensor to receive the external optical signal; and
the cover plate further comprises a third area (1013) a shielding structure (1015) is disposed on the third area, and the shielding structure is configured to isolate light between the optical device and the photoelectric sensor; wherein
the photoplethysmography sensor further comprises a light insulating plate (106) configured to isolate the optical device and the photoelectric sensor, the light insulating plate is disposed around the optical device, and one end of the light insulating plate is connected to the cover plate
**characterised in that** the shielding structure comprises a light-shielding ring, the light-shielding ring is disposed around the first area, the light-shielding ring is carved through a laser process, and the light-shielding ring is injected with a light-shielding material.

2. The photoplethysmography sensor according to claim 1, wherein the shielding structure comprises a groove, and the groove is disposed around the first area.

3. The photoplethysmography sensor according to claim 2, wherein a depth of the groove is 0.002 mm to 0.2 mm.

4. The photoplethysmography sensor according to claim 2 or 3, wherein an inner surface of the groove is provided with a grain.

5. The photoplethysmography sensor according to any one of claims 2 to 4, wherein the inner surface of the groove is covered with a coating for absorbing light.

6. The photoplethysmography sensor according to any one of claims 1 to 5, wherein the third area is covered with a coating for absorbing light, and the coating is located on a side, of the third area, facing the housing.

7. The photoplethysmography sensor according to claim 5 or 6, wherein the coating is black ink.

8. The photoplethysmography sensor according to any one of claims 1 to 7, wherein the housing comprises a bottom housing and a convex part, the bottom housing and the convex part are integrally formed, the cover plate is flat-shaped, the convex part is provided with a first opening, and the cover plate is clamped to the first opening.

9. The photoplethysmography sensor according to any one of claims 1 to 8, wherein the housing comprises a bottom housing, the bottom housing is provided with a second opening, the cover plate is convex, and the cover plate is clamped to the second opening.

10. The photoplethysmography sensor according to claim 9, wherein a recess is provided on a side, of the cover plate, facing the optical device, and the optical device is disposed in the recess.

11. The photoplethysmography sensor according to claim 10, wherein a side, of the cover plate, facing away from the optical device is provided with a planar face, and the planar face is opposite to the recess.

12. The photoplethysmography sensor according to claim 10 or 11, wherein the optical device is fastened in the recess through glue injection.

13. The photoplethysmography sensor according to any one of claims 1 to 12, wherein the second area comprises one or more light transmission windows, each light transmission window corresponds to one photoelectric sensor, and the light transmission windows are uniformly arranged around the first area.

14. The photoplethysmography sensor according to any one of claims 1 to 13, wherein a material of the housing is ceramic or plastic, and a material of the cover plate is sapphire or toughened glass.

## Patentansprüche

1. Fotoplethysmographiesensor, umfassend: ein Gehäuse (102), eine Abdeckplatte (101), eine optische Vorrichtung (103), die dazu konfiguriert ist, Licht nach außen zu emittieren, und einen fotoelektrischen Sensor (105), der dazu konfiguriert ist, ein externes optisches Signal zu empfangen, wobei
das Gehäuse und die Abdeckplatte einen geschlossenen Raum bilden und die optische Vorrichtung und der fotoelektrische Sensor in dem geschlossenen Raum untergebracht sind;
die Abdeckplatte einen ersten Bereich (1011), der durch die optische Vorrichtung dazu verwendet wird, das Licht nach außen zu emittieren, und einen zweiten Bereich (1012), der durch den fotoelektrischen Sensor dazu verwendet wird, das externe optische Signal zu empfangen, umfasst; und
die Abdeckplatte ferner einen dritten Bereich (1013) umfasst, eine Abschirmstruktur (1015) auf dem dritten Bereich angeordnet ist und die Abschirmstruktur dazu konfiguriert ist, Licht zwischen der optischen Vorrichtung und dem fotoelektrischen Sensor zu isolieren; wobei
der Fotoplethysmographiesensor ferner eine Lichtisolationsplatte (106) umfasst, die dazu konfiguriert ist, die optische Vorrichtung und den fotoelektrischen Sensor zu isolieren, die Lichtisolationsplatte um die optische Vorrichtung herum angeordnet ist und ein Ende der Lichtisolationsplatte mit der Abdeckplatte verbunden ist,
**dadurch gekennzeichnet, dass**
die Abschirmstruktur einen Lichtabschirmring umfasst, der Lichtabschirmring um den ersten Bereich herum angeordnet ist, der Lichtabschirmring durch einen Laserprozess herausgearbeitet ist und in den Lichtabschirmring ein Lichtabschirmmaterial eingespritzt ist.

2. Fotoplethysmographiesensor nach Anspruch 1, wobei die Abschirmstruktur eine Nut umfasst und die Nut um den ersten Bereich herum angeordnet ist.

3. Fotoplethysmographiesensor nach Anspruch 2, wobei eine Tiefe der Nut 0,002 mm bis 0,2 mm beträgt.

4. Fotoplethysmographiesensor nach Anspruch 2 oder 3, wobei eine Innenfläche der Nut mit einer Körnung versehen ist.

5. Fotoplethysmographiesensor nach einem der Ansprüche 2 bis 4, wobei die Innenfläche der Nut mit einer Beschichtung zum Absorbieren von Licht bedeckt ist.

6. Fotoplethysmographiesensor nach einem der Ansprüche 1 bis 5, wobei der dritte Bereich mit einer Beschichtung zum Absorbieren von Licht bedeckt ist und sich die Beschichtung auf einer Seite des dritten Bereichs befindet, die dem Gehäuse zugewandt ist.

7. Fotoplethysmographiesensor nach Anspruch 5 oder 6, wobei die Beschichtung schwarze Tinte ist.

8. Fotoplethysmographiesensor nach einem der Ansprüche 1 bis 7, wobei das Gehäuse ein Bodengehäuse und einen konvexen Teil umfasst, das Bodengehäuse und der konvexe Teil einstückig gebildet sind, die Abdeckplatte flach geformt ist, der konvexe Teil mit einer ersten Öffnung versehen ist und die Abdeckplatte an der ersten Öffnung festgeklemmt ist.

9. Fotoplethysmographiesensor nach einem der Ansprüche 1 bis 8, wobei das Gehäuse ein Bodengehäuse umfasst, das Bodengehäuse mit einer zweiten Öffnung versehen ist, die Abdeckplatte konvex ist und die Abdeckplatte an der zweiten Öffnung festgeklemmt ist.

10. Fotoplethysmographiesensor nach Anspruch 9, wobei eine Vertiefung auf einer Seite der Abdeckplatte bereitgestellt ist, die der optischen Vorrichtung zugewandt ist, und die optische Vorrichtung in der Vertiefung angeordnet ist.

11. Fotoplethysmographiesensor nach Anspruch 10, wobei eine Seite der Abdeckplatte, die von der optischen Vorrichtung abgewandt ist, mit einer ebenen Fläche versehen ist und die ebene Fläche der Vertiefung gegenüberliegt.

12. Fotoplethysmographiesensor nach Anspruch 10 oder 11, wobei die optische Vorrichtung durch Klebstoffeinspritzung in der Vertiefung befestigt ist.

13. Fotoplethysmographiesensor nach einem der Ansprüche 1 bis 12, wobei der zweite Bereich ein oder mehrere Lichtübertragungsfenster umfasst, jedes Lichtübertragungsfenster einem fotoelektrischen Sensor entspricht und die Lichtübertragungsfenster gleichmäßig um den ersten Bereich herum angeordnet sind.

14. Fotoplethysmographiesensor nach einem der Ansprüche 1 bis 13, wobei ein Material des Gehäuses Keramik oder Kunststoff ist und ein Material der Abdeckplatte Saphir oder gehärtetes Glas ist.

## Revendications

1. Capteur de photopléthysmographie, comprenant : un boîtier (102), une plaque de recouvrement (101), un dispositif optique (103), configuré pour émettre de la lumière vers l'extérieur, et un capteur photoélectrique (105), configuré pour recevoir un signal optique externe, dans lequel
le boîtier et la plaque de recouvrement forment un espace clos, et le dispositif optique et le capteur photoélectrique sont logés dans l'espace clos ;
la plaque de recouvrement comprend une première zone (1011) utilisée par le dispositif optique pour émettre la lumière vers l'extérieur et une deuxième zone (1012) utilisée par le capteur photoélectrique pour recevoir le signal optique externe ; et
la plaque de recouvrement comprend également une troisième zone (1013), une structure de protection (1015) est disposée sur la troisième zone, et la structure de protection est configurée pour isoler la lumière entre le dispositif optique et le capteur photoélectrique ; dans lequel
le capteur de photopléthysmographie comprend également une plaque isolante de lumière (106) configurée pour isoler le dispositif optique et le capteur photoélectrique, la plaque isolante de lumière est disposée autour du dispositif optique, et une extrémité de la plaque isolante de lumière est connectée à la plaque de recouvrement **caractérisée en ce que**
la structure de protection comprend un anneau de protection contre la lumière, l'anneau de protection contre la lumière est disposé autour de la première zone, l'anneau de protection contre la lumière est sculpté par un processus laser, et l'anneau de protection contre la lumière est injecté avec un matériau de protection contre la lumière.

2. Capteur de photopléthysmographie selon la revendication 1, dans lequel la structure de protection comprend une rainure, et la rainure est disposée autour de la première zone.

3. Capteur de photopléthysmographie selon la revendication 2, dans lequel une profondeur de la rainure est de 0,002 mm à 0,2 mm.

4. Capteur de photopléthysmographie selon la revendication 2 ou 3, dans lequel une surface intérieure de la rainure est pourvue d'un grain.

5. Capteur de photopléthysmographie selon l'une quelconque des revendications 2 à 4, dans lequel la surface intérieure de la rainure est recouverte d'un revêtement destiné à absorber la lumière.

6. Capteur de photopléthysmographie selon l'une quelconque des revendications 1 à 5, dans lequel la troisième zone est recouverte d'un revêtement destiné à absorber la lumière, et le revêtement est situé sur un côté de la troisième zone faisant face au boîtier.

7. Capteur de photopléthysmographie selon la revendication 5 ou 6, dans lequel le revêtement est de l'encre noire.

8. Capteur de photopléthysmographie selon l'une quelconque des revendications 1 à 7, dans lequel le boîtier comprend un boîtier inférieur et une partie convexe, le boîtier inférieur et la partie convexe sont formés d'un seul tenant, la plaque de recouvrement est de forme plate, la partie convexe est pourvue d'une première ouverture, et la plaque de recouvrement est serrée sur la première ouverture.

9. Capteur de photopléthysmographie selon l'une quelconque des revendications 1 à 8, dans lequel le boîtier comprend un boîtier inférieur, le boîtier inférieur est pourvu d'une seconde ouverture, la plaque de recouvrement est convexe, et la plaque de recouvrement est serrée sur la seconde ouverture.

10. Capteur de photopléthysmographie selon la revendication 9, dans lequel un évidement est pourvu sur un côté de la plaque de recouvrement, faisant face au dispositif optique, et le dispositif optique est disposé dans l'évidement.

11. Capteur de photopléthysmographie selon la revendication 10, dans lequel un côté de la plaque de recouvrement opposé au dispositif optique est pourvu d'une face plane, et la face plane est opposée à l'évidement.

12. Capteur de photopléthysmographie selon la revendication 10 ou 11, dans lequel le dispositif optique est fixé dans l'évidement par injection de colle.

13. Capteur de photopléthysmographie selon l'une quelconque des revendications 1 à 12, dans lequel la deuxième zone comprend une ou plusieurs fenêtres de transmission de lumière, chaque fenêtre de transmission de lumière correspond à un capteur photoélectrique, et les fenêtres de transmission de lumière sont disposées uniformément autour de la première zone.

14. Capteur de photopléthysmographie selon l'une quelconque des revendications 1 à 13, dans lequel un matériau du boîtier est de la céramique ou du plastique, et un matériau de la plaque de recouvrement est du saphir ou du verre trempé.
